# NEW EUROPEAN PATENT SPECIFICATION

(11) **EP 0 460 716 B2**
(45) Date of publication and mention of the opposition decision: **15.12.1999**
(45) Mention of the grant of the patent: 05.06.1996
(21) Application number: 91114001.0
(22) Date of filing: 06.01.1984
(51) Int. Cl.: C12N 15/81

(54) **Yeast expression systems with vectors having GAPDH promoters, and synthesis of HBsAg**
Expressionssysteme für Hefe mit Vektoren, die einen GAPDH-Promotor enthalten und Oberflächenantigensynthese von Hepatitis B
Système d'expression pour levure avec des vecteurs contenant un promoteur GAPDH et synthèse d'antigène de surface d'hépatite B

(30) Priority: 22.02.1983 US 468589
(43) Date of publication of application: 11.12.1991
(62) Divisional of application: 89106868.6
(73) Proprietor: CHIRON CORPORATION, Emeryville, California 94608 (US)
(72) Inventor: Burke, Rae Lyn, San Francisco, CA (US); Tekamp-Olson, Patricia, San Francisco, CA (US); Rosenberg, Steven, Oakland, CA (US); Valenzuela, Pablo D. T., San Francisco, CA (US)
(74) Representative: Hallybone, Huw George

(56) References cited:
- EP-A- 60 057
- EP-A- 72 318
- PROCEEDINGS OF THE NATIONAL ACADEMY OF SCIENCES USA, vol. 80, no. 1, 01 January 1983, Washington, DC (US); A. MIYANOHARA et al., p. 1

## Description

### BACKGROUND OF THE INVENTION

For maximal expression of foreign genes in microbial systems it is usually advantageous to employ homologous regulatory elements within the expression vector. Efficiency of expression (product formation) is believed to be a function of and proportional to the strength of the promoter employed. In addition, regulation of gene expression by nutritional factors under the control of the experimenter offers a further useful manipulatory tool. The glycolytic enzyme genes of yeast, e.g., those coding for glyceraldehyde-3-phosphate dehydrogenase (GAPDH) and pyruvate kinase (PyK), possess the above useful properties, i.e., high levels of expression (and thus by inference very efficient promoters) and susceptibility to regulation by components of the growth medium. For example, GAPDH can comprise as much as 5% of the dry weight of commercial baker's yeast (Krebs, E.G., J. Biol. Chem. (1953) 200:471) . Furthermore, these enzymes are also highly inducible. For example, when yeast cultures are shifted from growth on acetate to glucose, the activity of GAPDH increased up to 200-fold in proportion to the concentration of the sugar in the medium (Maitra, P.K. and Lobo, %., J. Biol. Chem. (1971) 246: 475). These results suggest that the transcriptional machinery of these genes is highly regulated, perhaps by the participation of DNA sequences present in the 5' non-coding flanking region of the genes.

This invention relates to the isolation, structure and the successful use in yeast expression plasmids of DNA fragments corresponding to the 5' non-coding regions of the regulatable yeast genes CAPDH. These fragments which contain DNA sequences with strong transcription-promoting activity are called "promoters". They are ideal components of DNA vectors for commercial production of large quantities of protein coded by foreign genes under their transcriptional control.

In addition, this invention encompasses yeast expression plasmids further comprising an appropriate terminator to a form a "cassette" of promoter-foreign gene-terminator. The presence of the terminator increases expression of the foreign DNA.

An early attempt to express foreign DNA in yeast failed (Beggs, J.D. et al., Nature (1980) 283:285). In this report, the hemoglobin DNA (inserted with its own promoter) was transcribed but the RNA was not spliced. A variety of explanations for this result are possible, e.g., an incorrect location for the initiation of transcription and/or the poor ability of yeast cells to carry out splicing of intervening sequences (introns).

Three GAPDH genes of yeast have been cloned (Holland, M.J. et al., Basic Life Science (1981) 19:291), but their promoters have not been used for constructing expression systems in yeast by recombinant DNA methods. The PyK gene has also been cloned, but by genetic complementation only (no structural studies performed) (Kawasaki, G. and Fraenel, D.G., Biochem. Biophys. Res. Comm. (1982) 108:1107). Other yeast promoters, e.g., that of alcohol dehydrogenase I (Valenzuela, P. et al., Nature (1982) 298:347 and Hitzeman, R.A. et al., Nature (1981) 293:717) and phosphoglycerate kinase (Tuite, M.F. et al., EMBO J. (1982) 1:603 and Hitzeman, R.A. et al., Science (1983) 219:620) have been linked to foreign genes to produce yeast expression but no terminators were used. The present invention provides new promoters for yeast expression systems and combines the advantages of highly expressive promoters with the enhanced expression found with appropriately ligated terminators.

A published European Patent Application (072 318) disclosed the construction of a yeast expression vector which, upon induction, expressed hepatitis B virus surface antigen (HBsAg) S-protein under control of the yeast alcohol dehydrogenase I (ADHI) promoter.

### BRIEF DESCRIPTION OF THE INVENTION

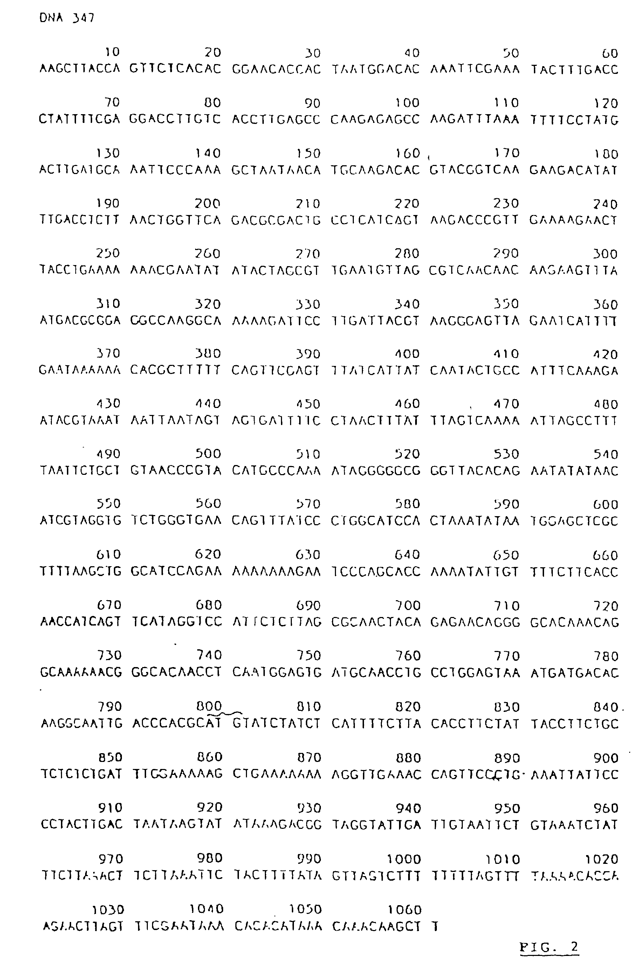

This invention relates to a method for expressing a DNA coding segment which encodes hepatitis B surface antigen or a portion thereof in yeast, comprising the steps of a) inserting said coding segment into a yeast expression vector to form a coding segment expression vector, and b) transforming yeast cells with the coding segment expression vector wherein said yeast expression vector comprises a promoter segment derived from a yeast glyceraldehyde-3-phosphate dehydrogenase promoter having the sequence shown in Figure 2 and having fewer than three codons from the 5' end of yeast glyceraldehyde-3-phosphate dehydrogenase, said promoter segment being in the correct orientation for transcription of the coding segment. The invention also relates to a method for the production of hepatitis B surface antigen or a portion thereof comprising the steps of a) culturing yeast cells transformed with a coding segment expression vector as defined above, and b) isolating hepatitis B surface antigen or a portion thereof.

Terminators may also be appropriately attached. The expression vector typically has a yeast replication origin and is capable of replicating in either type of cell. The expression vector, when used to transform yeast cells, will yield substantial amounts of the protein coded by the segment of foreign DNA.

### BRIEF DESCRIPTION OF THE DRAWINGS

Fig 1:Isolation and tailoring of a GAPDH promoter fragment.

Fig 2:DNA sequence of a GAPDH promoter fragment.

Fig 3:Construction of a yeast expression plasmid containing the GAPDH promoter.

### DETAILED DESCRIPTION OF THE INVENTION

In principle, yeast expression plasmids have particular advantages, including the following. Yeast can be grown in large-scale culture for commercial production by processes well-known in the art. In contrast, bacteria in large-scale culture are subject to the frequent problem of "phage-out". Yeast also appears to have much the same ability as mammalian cells to add carbohydrate groups to newly synthesized proteins, a capacity that bacteria do not have. Now that cDNA sequences are readily obtainable, the problem of expressing genes having introns is easily avoided.

The vectors of the present invention encompass promoters of unusually high efficiency. A promoter is defined herein as a DNA segment capable of functioning to initiate transcription of an adjoining DNA segment. Transcription is the synthesis of RNA (herein termed messenger RNA or mRNA), complementary to one strand of the DNA adjoining the promoter region. In eukaryotes, messenger RNA synthesis is catalyzed by an enzyme termed RNA polymerase II. The minimum essential elements of promoter function are the following: To provide a starting point for the initiation of transcription and to provide a binding site for RNA polymerase II near the start site permitting selection of the proper strand of DNA as a template for messenger RNA synthesis. In addition, a eukaryotic promoter functions to regulate the relative efficiency of transcription of coding segments under its control. An active promoter is one which elicits synthesis of relatively large amounts of mRNA complementary to a strand of the adjacent DNA coding segment.

The structural correlates of promoter function have not been clearly established. A promoter segment usually can be identified in nature as a region lying adjacent to the 5' end of a given structural gene. (References to the 5' and 3' ends of a gene will be understood to indicate the corresponding respective ends of mRNA transcribed therefrom, and these, in turn, will be understood to correlate with the NH₂- and -COOH termini of the encoded protein, respectively.) Comparisons of the nucleotide sequences of promoters for various genes from various species have revealed only a few short regions of nucleotide sequence similarity in common among them. Most notable of these is the "TATA Box, " a segment of about 5 to 10 nucleotides located generally about 70 to 230 nucleotides upstream from the site of transcription initiation, having a sequence generally resembling TATAA. For review of structural comparisons see Breathnach, R. and Chambon, P., Ann. Rev. of Biochem. (1981) 50 : 349. The TATA box is believed to function in initiation of transcription.

The foreing gene will be free or substantially free of condons form the normal structural gene associated with the promoter. Usually, the foreign gene will be joined to a non-coding 3'-end of the regulatory region encompassing the promoter, so as to be free of the amino acids at the N-terminus of endogenous gene naturally associated with the regulatory region. That is, fewer than about 3 codons (9 nucleotides) will be retained with the regulatory region when joined to the foreign gene.

The presence of the terminator sequence at the 3' end of the coding segment enhances expression. the effect is generally similar to the addition of rho factor to prokaryotic transcription systems, wherein the rate of the release of RNA polymerase is enhanced to produce an increase in the rate of reinitiation of transcription. It will be understood that, while the terminator sequences are not required for detectable expression of foreign DNA segments, it is preferable to appropriately link them to enhance expression. The terminator region may be naturally associated with the same or different structural gene as the promoter region.

The most appropriate DNA vector for the GAPDH construction of this invention is a shuttle vector. These vectors can "shuttle" between a bacterial strain, such as E. coli, and yeast, since they have a bacterial origin of replication and a yeast origin of replication, see, e.g., Ammerer, G. et al., Recombinant DNA, Proc. Third Cleveland Symposium Macromolecules (Walton, A.C., ed.), p. 185, Elsevier, Amsterdam (1981). A typical bacterial origin of replication is derived from, e.g., pBR322. The most useful yeast origin of replication is found in the extrachromosomal genetic element known as the 2 micron circle. In laboratory strains the 2 micron plasmid DNA is found in approximately 50 copies per cell and is stably maintained. For a review, see, for example, Curr. Topics Micro. Imm. (1982) 96:119. This yeast plasmid has also been sequenced (Hartley, J.L. et al., Nature (1980) 286:860).

Representative samples of the plasmids and host cells used in the constructions of this invention have been placed on deposit with the American Type Culture Collection, 12301 Parklawn Drive, Rockville, Maryland. Yeast cell transformant 2150-2-3/pHBS-56 GAP347/33 was placed on deposit on February 18, 1983 and has received ATCC Accession number 20665.

In the Examples that follow, many of the techniques, reactions and separation procedures are already well-known in the art. All enzymes, unless otherwise stated, are available from one or more commercial sources, such as New England Biolabs, Beverly, Massachusetts; Collaborative Research, Waltham, Massachusetts; Miles Laboratories, Elkhart, Indiana; Boehringer Biochemicals, Inc., Indianapolis, Indiana and Bethesda Research Laboratories, Rockville, Maryland. Buffers and reaction conditions for restriction enzyme digestion were used according to recommendations supplied by the manufacturer for each enzyme, unless otherwise indicated. Standard methodology for other enzyme reactions, gel electrophoresis separations and E. coli transformation may be found in Methods in Enzymology, (1979) 68. Transformation of yeast protoplasts can be carried out essentially as described by Beggs, Nature (1978) 275:104.

E. coli strains useful for transformation include X1776; K12 strain 294 (ATCC No. 31446); RR1 and HB101. Yeast strains XV610-8c having the genotype (a ade2 ade6 leu2 lys1 trp1 can1) and GM-3C-2, genotype: (Leu2 Trp1 His4 CYC1-1CYP3-1) (Faye, G. et al., Proc. Natl. Acad. Sci. (1981)78:2258) can be typically used for yeast transformations. It would be understood, however, that virtually any strain of yeast is useful for transformation. Bacteria can be grown and selected according to procedures described by Miller, J.H., Experiments in Molecular Genetics, Cold Spring Harbor Laboratory, Cold Spring Harbor, New York (1972). Yeast can be grown on the following media: YEPD containing 1% (w/v) yeast extract, 2% (w/v) peptone and (w/v) glucose; and, in the case of plating medium, 3% (w/v) agar. YNB plus CAA contains 6.7 grams of yeast nitrogen base (Difco Laboratories, Minneapolis, Minnesota), 10mg of adenine, 10mg of uracil, 5g casamino acids (CAA) (Difco), 20g glucose; and, in the case of plating media, 30g agar per liter. Selection for tryptophan prototrophy can be made on plates containing 6.7g yeast nitrogen base (lacking amino acids), supplemented for all growth requirements of the strain to be transformed except tryptophan.

### EXAMPLE 1

### Cloning of the yeast glyceraldehyde-3-phosphate dehydrogenase (GAPDH) gene.

A complementary DNA (cDNA) containing the yeast GAPDH coding sequences was prepared in the following manner:

PolyA+ RNA was isolated from yeast strain A364A. Double-stranded cDNA was synthesized using AMV reverse transcriptase and E. coli DNA polymerase I. Poly-dC-tails were added to the double-stranded cDNA molecule using deoxynucleotide terminal transferase. Poly-dC-tailed cDNA was annealed to poly-dG-tailed pBR322 and used to transform E. coli HB101. One thousand transformants were screened by colony hybridization to labeled PolyA+ RNA, and a subset further examined by restriction endonuclease mapping, and DNA sequencing. Three clones containing GAPDH sequences were isolated from the pool. One clone (pcGAP-9) contained an insert of about 1200 base pairs (bp) and was used for further work.

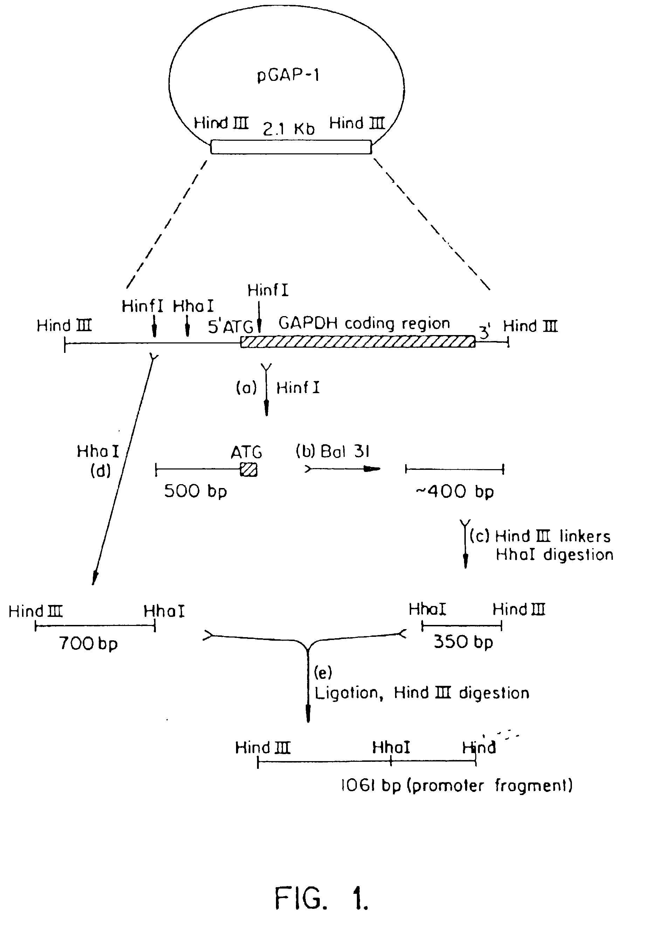

A yeast gene library was prepared by inserting fragments obtained after partial digestion of total yeast DNA with restriction endonuclease Sau3A into lambda phage Charon 28, according to Blattner, F.R. et al., Science (1977) 196: 161-169. Several fragments containing yeast GAPDH coding sequences were isolated by screening the phage library with labeled DNA from pcGAP-9. The yeast GAPDH gene of one of these clones was subcloned in pBR322 as a 2.1kb HindIII fragment (pGAP-1, see Fig.1) or as a 3.5kb BamHI fragment (pGAP-2). The GAPDH promoting-active fragments were isolated from these clones. The HindIII-HhaI fragment of about 800bp was ligated to the HhaI-HindIII fragment of about 350bp. The resulting 1061bp HindIII fragment was isolated by gel electrophoresis and cloned in pBR322, (pGAP-347), and the sequence determined (see Fig. 2).

### EXAMPLE 2

### Construction of yeast vectors containing the GAPDH promoter, active in the expression of HBsAg.

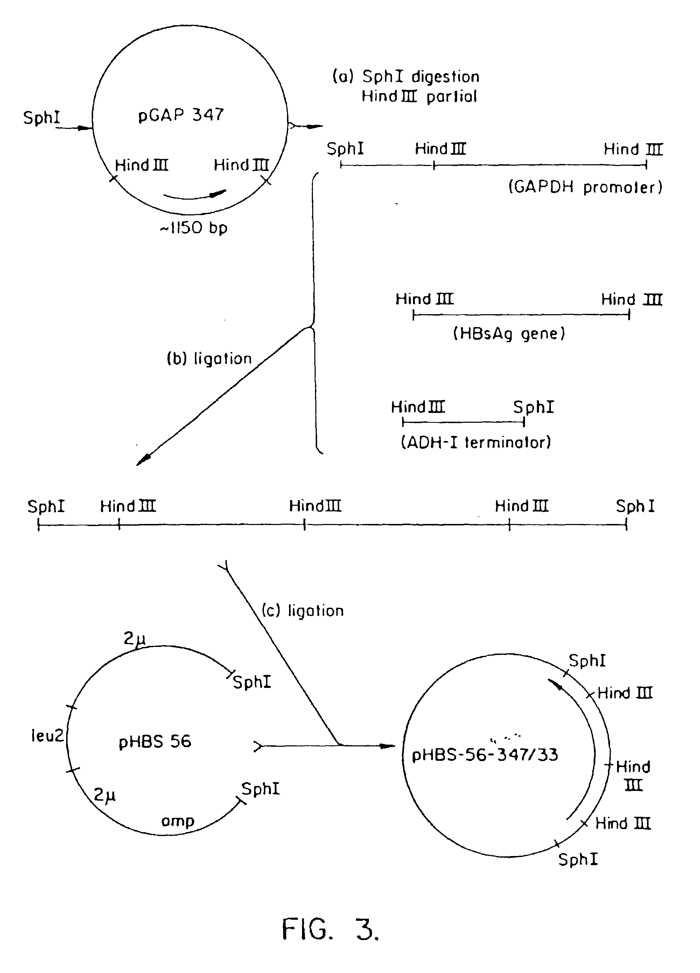

A plasmid vector (pHBS-56GAP347/33), for the expression of HBV surface antigen in yeast, using the GAPDH promoter fragment was constructed as depicted in Fig. 3.

Total digestion of pGAP-347 with SphI followed by partial digestion with HindIII yielded an approximately 1700bp SphI-HindIII fragment having about 1060bp of GAPDH promoter and about 530bp of pBR322. The 1700bp SphI-HindIII GAPDH promoter fragment was ligated with the 840bp HindIII-HindIII fragment (containing the HBsAg coding region, 26 bases of 5' non-coding region and 128bp of 3' non-coding region, obtained from pHBS-56) and then with the 350bp HindIII-SphI fragment containing the ADH-1 termination region (isolated from pHBS-56). The 2900bp SphI fragment (cassette) was isolated and cloned in pHBS-56 previously digested with SphI. The plasmid pHBS-56 (ATCC Accession No. 40047) has been described in a co-pending application (EPA No. 82.401473.2 published as no. 72318, of Regents of the University of California and contains the entire 2 micron plasmid, in addition to a region with the yeast leu2 gene and the amp resistance locus of pBR322. The resulting plasmid (pHBS-56GAP347/33) in which the promoter, gene and termination regions were in the proper orientations was isolated and used to transform yeast strain AB102 (MATa, pep 4-3, leu 2-3 leu2-112, ura 3-52, his 4-580, cir°) or strain 2150-2-3 (MATa, adel, leu2-04, cir°). Strain AB102 is derived from SF657-9c by curing of 2 micron plasmids. Strain 2150-2-3 is from the collection of Dr. Leland Hartwell at the University of Washington.

### EXAMPLE 3

### Synthesis of HBsAg in yeast under GAPDH promoter control (plasmid pHBS-56GAP347/33).

One hundred ml cultures of strain AB102 containing plasmid pHBS56-347/33 were grown to optical density at 650nm of 1. Cell-free lysates were prepared by agitation with glass beads and removal of cell debris by centrifugation. HBsAg was measured by the Abbott Ausriall radioimmunoassay and protein concentration was determined by the Coomassie blue binding method. The results are shown in Table 1. They indicate that the GAPDH promoter is about 5 times more effective than the ADH-1 promoter for protein product expression in yeast.

**Table 1**

| Synthesis of HBsAg in yeast | | | |
|---|---|---|---|
| (a) control from pHBS-56 (ADH-I promoter) | | | |
| Exp# | sAg (µg/ml) | protein (mg/ml) | Spec. Activity (µgsAg/mg protein) |
| 1 | 8.8 | 18 | 0.49 |
| 2 | 14 | 25 | 0.56 |
| 3 | 12.4 | 20 | 0.62 |

| (b) from pHBS-56GAP347/33 (GAPDH promoter) | | | |
|---|---|---|---|
| Exp# | sAg (µg/ml) | protein (mg/ml) | Spec. Activity (µgsAg/mg protein) |
| 1 | 36 | 14 | 2.6 |
| 2 | 35 | 12 | 2.9 |
| 3 | 37 | 12.5 | 3.0 |

Similar results were obtained by substituting yeast strain 2150-2-3 for yeast strain AB102 and repeating Example 3.

## Claims (Claims for the following Contracting State(s): BE, CH, DE, FR, GB, IT, LI, LU, NL, SE)

1. A method for expressing a DNA coding segment which encodes hepatitis B surface antigen or a portion thereof in yeast, comprising the steps of
a) inserting said coding segment into a yeast expression vector to form a coding segment expression vector, and
b) transforming yeast cells with the coding segment expression vector
wherein said yeast expression vector comprises a promoter segment derived from a yeast glyceraldehyde-3-phosphate dehydrogenase promoter having the sequence shown in Figure 2 and having fewer than three codons from the 5' end of yeast glyceraldehyde-3-phosphate dehydrogenase, said promoter segment being in the correct orientation for transcription of the coding segment.

2. A method for the production of hepatitis B surface antigen or a portion thereof comprising the steps of
a) culturing yeast cells transformed with a coding segment expression vector comprising a DNA coding segment encoding hepatitis B surface antigen or a portion thereof, and
b) isolating hepatitis B surface antigen or a portion thereof
wherein said coding segment expression vector comprises a promoter segment derived from a yeast glyceraldehyde-3-phosphate dehydrogenase promoter having the sequence shown in Figure 2 and having fewer than three codons from the 5' end of yeast glyceraldehyde-3-phosphate dehydrogenase, said promoter segment being in the correct orientation for transcription of the coding segment.

3. A method according to claim 1 or 2, wherein said yeast expression vector further comprises a terminator attached to the 3' end of the inserted DNA coding segment.

4. A method according to claim 3, wherein said terminator comprises the yeast alcohol dehydrogenase I terminator.

5. A method according to claim 3, wherein said terminator comprises the yeast GAPDH terminator.

6. A method according to claim 3, wherein said terminator comprises the yeast PyK terminator.

7. A method according to any of one of claims 1 to 6, wherein said yeast expression vector further comprises a yeast cell replication origin and is capable of replicating in a yeast cell.

8. A method according to any of claims 1 to 6, wherein said yeast expression vector further comprises a bacterial cell replication origin and is capable of replicating in a bacterial cell.

9. A method according to any of claims 1 to 6, wherein said yeast expression vector further comprises a yeast cell replication origin and a bacterial cell replication origin and is capable of replicating in yeast cells or bacterial cells.

10. A method according to any of one of claims 1 to 6, wherein said yeast expression vector further comprises yeast two micron plasmid DNA or a portion thereof.

## Claims (Claims for the following Contracting State(s): AT)

1. A method for the production of hepatitis B surface antigen or a portion thereof comprising the steps of
a) culturing yeast cells transformed with a coding segment expression vector comprising a DNA coding segment encoding hepatitis B surface antigen or a portion thereof, and
b) isolating hepatitis B surface antigen or a portion thereof
wherein said coding segment expression vector comprises a promoter segment derived from a yeast glyceraldehyde-3-phosphate dehydrogenase promoter having the sequence shown in Figure 2 and having fewer than three codons from the 5' end of yeast glyceraldehyde-3-phosphate dehydrogenase, said promoter segment being in the correct orientation for transcription of the coding segment.

2. A method according to claim 1, wherein said yeast expression vector further comprises a terminator attached to the 3' end of the inserted DNA coding segment.

3. A method according to claim 2, wherein said terminator comprises the yeast alcohol dehydrogenase I terminator.

4. A method according to claim 2, wherein said terminator comprises the yeast GAPDH terminator.

5. A method according to claim 2, wherein said terminator comprises the yeast PyK terminator.

6. A method according to any of one of claims 1 to 5, wherein said yeast expression vector further comprises a yeast cell replication origin and is capable of replicating in a yeast cell.

7. A method according to any of claims 1 to 5, wherein said yeast expression vector further comprises a bacterial cell replication origin and is capable of replicating in a bacterial cell.

8. A method according to any of claims 1 to 5, wherein said yeast expression vector further comprises a yeast cell replication origin and a bacterial cell replication origin and is capable of replicating in yeast cells or bacterial to cells.

9. A method according to any of one of claims 1 to 5, wherein said yeast expression vector further comprises yeast two micron plasmid DNA or a portion thereof.

## Patentansprüche (Patentansprüche für folgende(n) Vertragsstaat(en): BE, CH, DE, FR, GB, IT, LI, LU, NL, SE)

1. Methode zur Expression eines DNS-Codierungssegments, das für ein Hepatitis B-Oberflächenantigen oder für ein Teilbereich desselben in Hefe codiert, die die Etappen
a) der Insertion des Codierungssegments in einen Hefe-Expressionsvektor um einen Codierungssegment-Expressionsvektor zu bilden, und
b) der Transformation von Hefezellen mit dem Codierungssegment-Expressionsvektor,
beinhaltet,
worin der Hefe-Expressionsvektor ein Promotorsegment enthält welches von einem Glyceraldehyd-3-phosphat-dehydrogenase-Promotor einer Hefe abgeleitet ist, der die in der Figur 2 gezeigte Sequenz aufweist und weniger als drei Codone vom 5'-Ende der Hefe-Glyceraldehyd-3-phosphat-dehydrogenase aufweist, wobei das Promotorsegment sich in der für die Transkription des Codierungssegments richtigen Orientierung befindet.

2. Methode zur Produktion des Hepatitis B-Oberflächenantigens oder eines Teilbereichs desselben, die die Etappen
a)der Kultur von Hefezellen welche mittels einem Codierungssegment-Expressionsvektor transformiert wurden, der ein DNS-Codierungssegment enthält das für ein Hepatitis B-Oberflächenantigen oder für ein Teilbereich desselben codiert, und
b) die Isolierung des Hepatitis B-Oberflächenantigens oder eines Teilbereichs desselben
beinhaltet,
worin der Codierungssegment-Expressionsvektor ein Promotorsegment enthält welches von einem Glyceraldehyd-3-phosphat-dehydrogenase-Promotor einer Hefe abgeleitet ist, der die in der Figur 2 gezeigte Sequenz aufweist und weniger als drei Codone vom 5'-Ende der Hefe-Glyceraldehyd-3-phosphat-dehydrogenase aufweist, wobei das Promotorsegment sich in der für die Transkription des Codierungssegments richtigen Orientierung befindet.

3. Methode nach Anspruch 1 oder 2, worin der Hefe-Expressionsvektor ferner einen am 3'-Ende des eingesetzten DNS-Codierungssegments angebundenen Terminator enthält.

4. Methode nach Anspruch 3, worin der Terminator den Hefe-Alkohol-dehydrogenase-1-Terminator enthält.

5. Methode nach Anspruch 3, worin der Terminator den Hefe-GAPDH-Terminator enthält.

6. Methode nach Anspruch 3, worin der Terminator den Hefe-PyK-Terminator enthält.

7. Methode nach einem der Ansprüche 1 bis 6, worin der Hefe-Expressionsvektor ferner ein Replikationsursprung aus einer Hefezelle enthält und fähig ist sich in einer Hefezelle zu replizieren.

8. Methode nach einem der Ansprüche 1 bis 6, worin der Hefe-Expressionsvektor ferner ein Replikationsursprung aus einer Bakterienzelle enthält und fähig ist sich in einer Bakterienzelle zu replizieren.

9. Methode nach einem der Ansprüche 1 bis 6, worin der Hefe-Expressionsvektor ferner ein Replikationsursprung aus einer Hefezelle oder ein Replikationsursprung aus einer Bakterienzelle enthält und fähig ist sich in Hefezellen oder in Bakterienzellen zu replizieren.

10. Methode nach einem der Ansprüche 1 bis 6, worin der Hefe-expressionsvektor ferner eine Zwei-Mikron-Hefe-Plasmid-DNS oder ein Teilbereich derselben enthält.

## Patentansprüche (Patentansprüche für folgende(n) Vertragsstaat(en): AT)

1. Methode zur Produktion des Hepatitis B-Oberflächenantigens oder eines Teilbereichs desselben, die die Etappen
a) der Kultur von Hefezellen welche mittels einem Codierungssegment-Expressionsvektor transformiert wurden, der ein DNS-Codierungssegment enthält das für ein Hepatitis B-Oberflächenantigen oder für ein Teilbereich desselben codiert, und
b) die Isolierung des Hepatitis B-Oberflächenantigens oder eines Teilbereichs desselben
beinhaltet,
worin der Codierungssegment-Expressionsvektor ein Promotorsegment enthält welches von einem Glyceraldehyd-3-phosphat-dehydrogenase-Promotor einer Hefe abgeleitet ist, der die in der Figur 2 gezeigte Sequenz aufweist und weniger als drei Codone vom 5'-Ende der Hefe-Glyceraldehyd-3-phosphat-dehydrogenase aufweist, wobei das Promotorsegment sich in der für die Transkription des Codierungssegments richtigen Orientierung befindet.

2. Methode nach Anspruch 1, worin der Hefe-Expressionsvektor ferner einen am 3'-Ende des eingesetzten DNS-Codierungssegments angebundenen Terminator enthält.

3. Methode nach Anspruch 2, worin der Terminator den Hefe-Alkohol-dehydrogenase-l-Terminator enthält.

4. Methode nach Anspruch 2, worin der Terminator den Hefe-GAPDH-Terminator enthält.

5. Methode nach Anspruch 2, worin der Terminator den Hefe-PyK-Terminator enthält.

6. Methode nach einem der Ansprüche 1 bis 5, worin der Hefe-Expressionsvektor ferner ein Replikationsursprung aus einer Hefezelle enthält und fähig ist sich in einer Hefezelle zu replizieren.

7. Methode nach einem der Ansprüche 1 bis 5, worin der Hefe-Expressionsvektor ferner ein Replikationsursprung aus einer Bakterienzelle enthält und fähig ist sich in einer Bakterienzelle zu replizieren.

8. Methode nach einem der Ansprüche 1 bis 5, worin der Hefe-Expressionsvektor ferner ein Replikationsursprung aus einer Hefezelle oder ein Replikationsursprung aus einer Bakterienzelle enthält und fähig ist sich in Hefezellen oder in Bakterienzellen zu replizieren.

9. Methode nach einem der Ansprüche 1 bis 5, worin der Hefe-Expressionsvektor ferner eine Zwei-Mikron-Hefe-Plasmid-DNS oder ein Teilbereich derselben enthält.

## Revendications (Revendications pour l'(les) Etat(s) contractant(s) suivant(s): BE, CH, DE, FR, GB, IT, LI, LU, NL, SE)

1. Méthode pour l'expression d'un segment codant d'ADN qui code pour un antigène de surface d'hépatite B ou une portion de celui-ci dans des levures, comprenant les étapes de
a) insertion dudit segment codant dans un vecteur d'expression de levure de façon à former un vecteur d'expression de segment codant, et
b) transformation de cellules de levure par le vecteur d'expression de segment codant
dans laquelle ledit vecteur d'expression de levure comprend un segment de promoteur dérivé d'un promoteur glycéraldéhyde-3-phosphate déshydrogénase de levure présentant la séquence montrée à la Figure 2 et présentant moins de trois codons de l'extrémité 5' de la glycéraldéhyde-3-phosphate déshydrogénase de levure, ledit segment de promoteur se trouvant dans l'orientation correcte pour la transcription du segment codant.

2. Méthode pour la production d'antigène de surface d'hépatite B ou d'une portion de celui-ci, comprenant les étapes de
a) culture de cellules de levure transformées par un vecteur d'expression de segment codant comprenant un segment codant d'ADN codant pour un antigène de surface d'hépatite B ou une portion de celui-ci, et
b) isolement de l'antigène de surface d'hépatite B ou d'une portion de celui-ci dans laquelle ledit vecteur d'expression de segment codant comprend un segment de promoteur dérivé d'un promoteur glycéraldéhyde-3-phosphate déshydrogénase de levure présentant la séquence montrée à la Figure 2 et présentant moins de trois codons de l'extrémité 5' de la glycéraldéhyde-3-phosphate déshydrogénase de levure, ledit segment de promoteur se trouvant dans l'orientation correcte pour la transcription du segment codant.

3. Méthode selon la revendication 1 ou 2, dans laquelle ledit vecteur d'expression de levure comprend en outre un terminateur attaché à l'extrémité 3' du segment codant d'ADN inséré.

4. Méthode selon la revendication 3, dans laquelle ledit terminateur comprend le terminateur de l'alcool déshydrogénase I de levure.

5. Méthode selon la revendication 3, dans laquelle ledit terminateur comprend le terminateur GAPDH de levure.

6. Méthode selon la revendication 3, dans laquelle ledit terminateur comprend le terminateur PyK de levure.

7. Méthode selon l'une quelconque des revendications 1 à 6, dans laquelle ledit vecteur d'expression de levure comprend en outre une origine de réplication de cellule de levure et est capable de se répliquer dans une cellule de levure.

8. Méthode selon l'une quelconque des revendications 1 à 6, dans laquelle ledit vecteur d'expression de levure comprend en outre une origine de réplication de cellule bactérienne et est capable de se répliquer dans une cellule bactérienne.

9. Méthode selon l'une quelconque des revendications 1 à 6, dans laquelle ledit vecteur d'expression de levure comprend en outre une origine de réplication de cellule de levure et une origine de réplication de cellule bactérienne et est capable de se répliquer dans des cellules de levure ou dans des cellules bactériennes.

10. Méthode selon l'une quelconque des revendications 1 à 6, dans laquelle ledit vecteur d'expression de levure comprend en outre un ADN de plasmide de deux microns de levure ou une portion de celui-ci.

## Revendications (Revendications pour l'(les) Etat(s) contractant(s) suivant(s): AT)

1. Méthode pour la production d'antigène de surface d'hépatite B ou d'une portion de celui-ci, comprenant les étapes de
a) culture de cellules de levure transformées par un vecteur d'expression de segment codant comprenant un segment codant d'ADN codant pour un antigène de surface d'hépatite B ou une portion de celui-ci, et
b) isolement de l'antigène de surface d'hépatite B ou d'une portion de celui-ci dans laquelle ledit vecteur d'expression de segment codant comprend un segment de promoteur dérivé d'un promoteur glycéraldéhyde-3-phosphate déshydrogénase de levure présentant la séquence montrée à la Figure 2 et présentant moins de trois codons de l'extrémité 5' de la glycéraldéhyde-3-phosphate déshydrogénase de levure, ledit segment de promoteur se trouvant dans l'orientation correcte pour la transcription du segment codant.

2. Méthode selon la revendication 1, dans laquelle ledit vecteur d'expression de levure comprend en outre un terminateur attaché à l'extrémité 3' du segment codant d'ADN inséré.

3. Méthode selon la revendication 2, dans laquelle ledit terminateur comprend le terminateur de l'alcool déshydrogénase I de levure.

4. Méthode selon la revendication 2, dans laquelle ledit terminateur comprend le terminateur GAPDH de levure.

5. Méthode selon la revendication 2, dans laquelle ledit terminateur comprend le terminateur PyK de levure.

6. Méthode selon l'une quelconque des revendications 1 à 5, dans laquelle ledit vecteur d'expression de levure comprend en outre une origine de réplication de cellule de levure et est capable de se répliquer dans une cellule de levure.

7. Méthode selon l'une quelconque des revendications 1 à 5, dans laquelle ledit vecteur d'expression de levure comprend en outre une origine de réplication de cellule bactérienne et est capable de se répliquer dans une cellule bactérienne.

8. Méthode selon l'une quelconque des revendications 1 à 5, dans laquelle ledit vecteur d'expression de levure comprend en outre une origine de réplication de cellule de levure et une origine de réplication de cellule bactérienne et est capable de se répliquer dans des cellules de levure ou dans des cellules bactériennes.

9. Méthode selon l'une quelconque des revendications 1 à 5, dans laquelle ledit vecteur d'expression de levure comprend en outre un ADN de plasmide de deux microns de levure ou une portion de celui-ci.
